# EUROPEAN PATENT APPLICATION

(11) **EP 4 206 185 A1**
(43) Date of publication of application: **05.07.2023**
(21) Application number: 21858490.2
(22) Date of filing: 28.07.2021
(51) Int. Cl.: C07C 255/23, A61L 24/04, A61L 24/00, A61K 49/04

(54) **NOVEL CYANOACRYLATE DERIVATIVE AND COMPOSITION FOR EMBOLIC MATERIAL CONTAINING SAME**

(30) Priority: 19.08.2020 KR 20200103788
(71) Applicant: Seoul National University R & DB Foundation, Seoul 08826 (KR); Seoul National University Hospital, Seoul 03080 (KR)
(72) Inventor: AHN, Cheol Hee, Seoul 04385 (KR); JEON, Seong Ik, Seoul 08840 (KR); JAE, Hwan Jun, Seoul 06001 (KR)
(74) Representative: Schmitt-Nilson Schraud Waibel Wohlfrom Patentanwälte Partnerschaft mbB
(86) International application number: PCT/KR2021/009813
(87) International publication number: WO 2022/039403

(57) **Abstract**

The present application relates to a novel cyanoacrylate derivative, and a composition for an embolic material, comprising the same.

## Description

### [Technical Field]

The present application relates to a novel cyanoacrylate derivative, and a composition for an embolic material, comprising the same.

### [Background Art]

Intravascular embolization refers to a procedure in which blood vessels in the target area are blocked with an embolic material to prevent additional bleeding if there is a risk of internal bleeding due to blood vessel rupture caused by the formation of malformed blood vessels, or if, due to internal and external factors, blood vessels existing in the liver, digestive organs, etc. are ruptured and bleeding occurs. Embolization is applied to diseases such as gastric varices, cerebral arteriovenous malformations, and aneurysms, and has advantages in that the procedure time is short, open surgery is unnecessary, and the recovery period is shortened.

Intravascular embolization is carried out by inserting a catheter through the femoral artery, approaching it to lesional vessels, then blocking them using liquid embolic materials which are rapidly solidified upon contacting blood. Since the procedure is performed without open surgery, it is common to monitor injection sites of embolic materials in real time through X-ray imaging devices. Therefore, the embolic materials must inevitably have radiopacity or be mixed with an X-ray contrast agent before use.

In the case of cyanoacrylate-based liquid embolic materials, which are currently widely used as embolic materials, they can effectively block target lesional blood vessels as they react with substances in the blood and rapidly polymerized to form solid substances insoluble in blood. However, since they do not have any radiopacity themselves, and their solidification rate is too fast, there are problems in that target lesional blood vessels cannot be accurately occluded, and they are polymerized inside the catheter during the procedure so that there is a risk of catheter blockage, and the adhesion force is too strong so that the risk of catheter entrapment within the blood vessel is high.

When the liquid embolic material does not have its own radiopacities, it is essential to mix it with radiopaque oils before use, so that there is a problem in that it is difficult to secure reproducibility depending on an operator. Moreover, when mixing, there is a risk that the liquid embolic material and the X-ray contrast agent are not sufficiently mixed or phase separation occurs so that there is a risk of causing a difference between the blood vessels where the actual embolization occurs and the area contrasted, which causes occlusion of normal blood vessels, not target lesional blood vessels, so that there are problems that may lead to serious medical accidents such as necrosis of normal tissues.

Meanwhile, in the case of an liquid embolic materials composed of polymers dissolved in an organic solvent, it is precipitated to solids inside blood vessels due to its low solubility in blood, blocking the blood flow. The embolic material has a low solidification rate and relieved adhesion to the catheter compared to cyanoacrylate-based liquid embolic materials, but has problems in that it does not have its own radiopacities, and there is a risk of the occurrence of toxicity and side effects due to the organic solvent.

Therefore, it is required to develop a new embolic material which does not require mixing of the contrast agent by having its own radiopacities, has low adhesion to the catheter in order to prevent the catheter entrapment within the blood vessel, has a mechanical strength that can withstand blood flow in order to effectively occlude the target lesional blood vessels, has a solidification rate appropriately adjusted in order to accurately occlude target lesional blood vessels, and does not use an organic solvent.

Korean Patent Publication No. 10-2010-0106966 discloses a single vial formulation of cyanoacrylate for medical use. Specifically, it relates to a medical cyanoacrylate stably storable in a single container by using a high-purity monomeric starting material formed of a more viscous oligomer and combining the material with a plasticizer and an inhibitor, and a method for producing the same. However, a composition for an embolic material, containing iodine and fluorine is not mentioned.

### [Disclosure]

### [Technical Problem]

The present application is to solve the problems of the conventional art described above, and an object of the present application is to provide a cyanoacrylate derivative, and a composition for an embolic material, comprising the same.

Another object of the present application is to provide an embolic material comprising the composition.

However, the technical problem to be achieved by the embodiments of the present application is not limited to the technical problems described above, and other technical problems may exist.

### [Technical Solution]

As a technical means for achieving the above technical problem, a first aspect of the present application provides a compound represented by Formula 1 below.

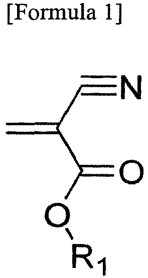

(In Formula 1, R₁ is a linear or branched C₁-C₁₀ alkyl in which one or more hydrogen atoms are substituted with iodine atoms).

According to one embodiment of the present application, the compound may be a compound represented by any one structural formula of Formulas 2 to 4 below, but is not limited thereto.

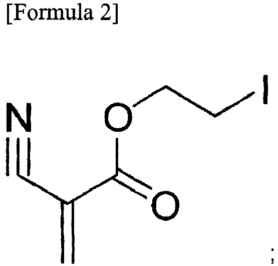

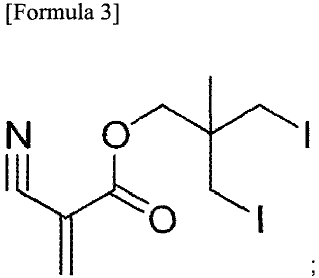

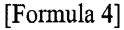

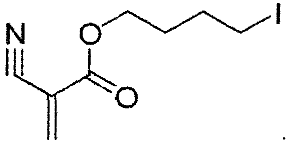

According to one embodiment of the present application, R₁ may be a linear or branched C₁-C₁₀ alkyl in which one or more hydrogen atoms are substituted with fluorine atoms, but is not limited thereto.

According to one embodiment of the present application, the compound may be a compound represented by any one structural formula of Formulas 5 and 6 below, but is not limited thereto.

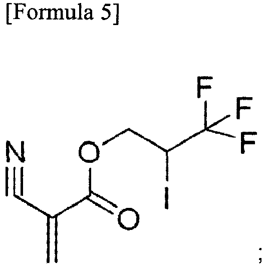

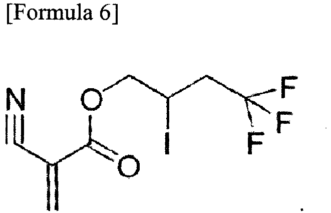

According to one embodiment of the present application, R₁ may be one in which one or more hydrogen atoms are substituted with a substituent represented by Formula 7 below, but is not limited thereto.

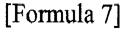

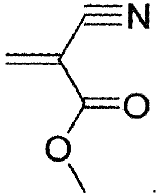

According to one embodiment of the present application, the compound may be one in which the substituent represented by Formula 7 above forms a symmetrical structure, but is not limited thereto.

According to one embodiment of the present application, the compound may be a compound represented by Formulas 8 to 9 below, but is not limited thereto.

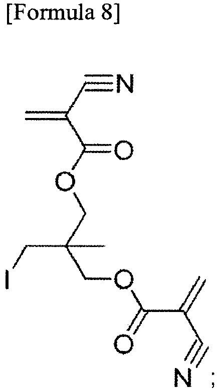

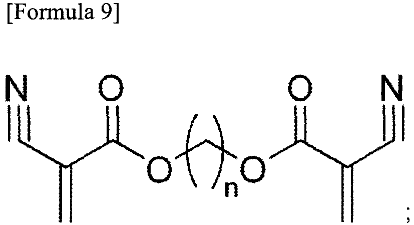

(In Formula 9, n is 1 to 15 linear or branched alkyls).

A second aspect of the present application provides a composition for an embolic material, comprising the compound according to the first aspect of the present application and a fatty acid ester oil.

According to one embodiment of the present application, the fatty acid ester oil may be a saturated or unsaturated fatty acid ester oil including one selected from the group consisting of a C₄-C₈ lower fatty acid ester oil, a C₈-C₁₂ medium fatty acid ester oil, a C₁₄-C₂₆ higher fatty acid ester oil, and combinations thereof, but is not limited thereto.

According to one embodiment of the present application, the fatty acid ester oil may include one selected from the group consisting of ethyl palmitate, ethyl oleate, ethyl linoleate, ethyl stearate, ethyl decanoate, lipiodol, and combinations thereof, but is not limited thereto.

According to one embodiment of the present application, the compound and the fatty acid ester oil may be contained at a volume ratio of 10:1 to 1:10, but is not limited thereto.

According to one embodiment of the present application, the composition for an embolic material may further comprise a compound represented by Formula 10 below, but is not limited thereto.

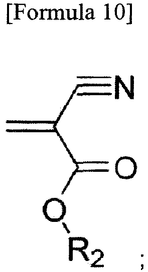

(In Formula 10, R₂ is a linear or branched C₁-C₁₀ alkyl in which one or more hydrogen atoms are substituted with fluorine atoms).

According to one embodiment of the present application, R₂ may include one selected from the group consisting of 2-fluoroethyl, 3-fluoroethyl, 2,2,2-trifluoroethyl, 3,3,3-trifluoropropyl, 2,2,3,3,3-pentafluoropropyl, and combinations thereof, but is not limited thereto.

According to one embodiment of the present application, the compound represented by Formula 10 above may be contained at a volume ratio of 10% to 50%, but is not limited thereto.

According to one embodiment of the present application, the composition for an embolic material may have an iodine content of 100 mgI/mL to 1,500 mgI/mL, but is not limited thereto.

According to one embodiment of the present application, the composition for an embolic material may further comprise one selected from the group consisting of inhibitors, accelerators, and combinations thereof to control the solidification rate, but is not limited thereto.

According to one embodiment of the present application, the inhibitor may be contained at a weight ratio of 1% to 30%, but is not limited thereto.

According to one embodiment of the present application, the inhibitor may include one selected from the group consisting of carboxylic acid-containing small molecular substances, acetic acid, phosphoric acid, sulfur dioxide, hydroquinone, carboxylic acid-containing fatty acids, and combinations thereof, but is not limited thereto.

According to one embodiment of the present application, the carboxylic acid-containing fatty acids may include one selected from the group consisting of oleic acid, linolenic acid, palmitic acid, and combinations thereof, but are not limited thereto.

According to one embodiment of the present application, the accelerator may be contained at a weight ratio of 10% to 30%, but is not limited thereto.

According to one embodiment of the present application, the accelerator may include one selected from the group consisting of hydroxyl group-containing small molecular substances, ethyl alcohol, butyl alcohol, 1,4-butanediol, and combinations thereof, but is not limited thereto.

According to one embodiment of the present application, the composition for an embolic material may have a solidification rate of 1 second to 30 seconds, but is not limited thereto.

The solidification rate of the composition for an embolic material may be adjusted within an error range of 1 second, but is not limited thereto.

The above-described problem solving means are merely exemplary and should not be construed as intended to limit the present application. In addition to the exemplary embodiments described above, additional embodiments may exist in the drawings and detailed description of the invention.

### [Advantageous Effects]

According to the above-described problem solving means of the present application, the cyanoacrylate derivative according to the present application contains an iodine atom, thereby having its own radiopacities. Therefore, since pre-mixing of the contrast agent is not necessary, it can be usefully used as an embolic material that can secure reproducibility operator-independently.

Further, since the cyanoacrylate-based compound according to the present application also contains a fluorine atom, its adhesion to the catheter is alleviated. Therefore, the compound can be usefully used as an embolic material that solves the problem of intravascular catheter entrapment.

Further, the composition for an embolic material according to the present application, also comprises the fatty acid ester, thereby appropriately adjusting the solidification rate. Therefore, the composition for an embolic material according to the present application can be usefully used as an embolic material capable of accurately occluding target blood vessels.

Further, the composition for an embolic material according to the present application has mechanical strength that can withstand blood flow, thereby enabling lesional blood vessels to be effectively occluded, and does not use an organic solvent, thereby enabling it to be usefully used as an embolic material without a risk of the occurrence of toxicity and side effects caused by the organic solvent.

However, the effects obtainable from the present application are not limited to the effects described above, and other effects may exist.

### [Description of Drawings]

FIG. 1 is a nuclear magnetic resonance (NMR) spectrum of a compound prepared in one Example of the present application.
FIG. 2 is a nuclear magnetic resonance (NMR) spectrum of a compound prepared in one Example of the present application.
FIG. 3 is a nuclear magnetic resonance (NMR) spectrum of a compound prepared in one Example of the present application.
FIG. 4 is a nuclear magnetic resonance (NMR) spectrum of a compound prepared in one Example of the present application.
FIG. 5 is a nuclear magnetic resonance (NMR) spectrum of a compound prepared in one Example of the present application.
FIG. 6 is a nuclear magnetic resonance (NMR) spectrum of a compound prepared in one Example of the present application.
FIG. 7 is a nuclear magnetic resonance (NMR) spectrum of a compound prepared in one Example of the present application.
FIG. 8 is nuclear magnetic resonance (NMR) spectra of compounds prepared in one Example of the present application.
FIG. 9 is an image showing the result of gel permeation chromatography (GPC) analysis of the compound prepared in one Example of the present application.
FIG. 10 is a graph showing the times required for solidification of the compounds prepared in one Example of the present application upon contact with blood.
FIG. 11 is a graph showing the results of measuring the catheter adhesion of the compounds prepared in one Example of the present application.
FIG. 12 is X-ray images showing the results of measuring the radiopacities under in vitro conditions of the compounds according to one Example and Comparative Example of the present application.
FIG. 13 is images showing the results of measuring the radiopacities using the compounds prepared in one Example of the present application.
FIG. 14 is images showing the results of measuring the radiopacities and embolic effects under in vivo conditions using a compound prepared in one Example of the present application.
FIG. 15 is CT images of the renal artery taken with the passage of time after performing embolization using a compound prepared in one Example of the present application.
FIG. 16 is CT images and histopathology images taken after performing embolization using a compound prepared in one Example of the present application.
FIG. 17 is images showing the results of measuring the radiopacities and embolic effects under in vivo conditions after performing embolization using a compound prepared in one Example of the present application.

### [Best Mode]

Hereinafter, embodiments of the present application will be described in detail with reference to the accompanying drawings so that those with ordinary skill in the art to which the present application pertains will easily be able to implement the present application.

However, the present application may be implemented in various different forms and is not limited to the embodiments described herein. Further, parts irrelevant to the description are omitted in order to clearly describe the present application in the drawings, and similar reference numerals are attached to similar parts throughout the specification.

In the whole specification of the present application, when a part is said to be "connected" with other part, it not only includes a case that the part is "directly connected" to the other part, but also includes a case that the part is "electrically connected" to the other part with another element being interposed therebetween.

In the whole specification of the present application, when any member is positioned "on", "over", "above", "beneath", "under", and "below" other member, this not only includes a case that the any member is brought into contact with the other member, but also includes a case that another member exists between two members.

In the whole specification of the present application, if a prescribed part "includes" a prescribed element, this means that another element can be further included instead of excluding other elements unless any particularly opposite description exists.

When unique manufacture and material allowable errors of numerical values are suggested to mentioned meanings of terms of degrees used in the present specification such as "about", "substantially", etc., the terms of degrees are used in the numerical values or as a meaning near the numerical values, and the terms of degrees are used to prevent that an unscrupulous infringer unfairly uses a disclosure content in which exact or absolute numerical values are mentioned to help understanding of the present application. Further, in the whole specification of the present application, "a step to do ~ " or "a step of ~ " does not mean "a step for ~ ".

In the whole specification of the present application, a term of "a com bination thereof' included in a Markush type expression, which means a mixt ure or combination of one or more selected from the group consisting of cons tituent elements described in the Markush type expression, means including on e or more selected from the group consisting of the constituent elements.

In the whole specification of the present application, description of "A and/or B" means "A, B, or A and B".

In the whole specification of the present application, a term "alkyl" may include a linear or branched, saturated or unsaturated C₁-C₆ alkyl, and may include, for example, methyl, ethyl, propyl, butyl, pentyl, hexyl, or all possible isomers thereof, but may not be limited thereto.

Hereinafter, a novel cyanoacrylate derivative according to the present application, and a composition for an embolic material, comprising the same, will be described in detail with reference to embodiments, Examples, and drawings. However, the present application is not limited to such embodiments, Examples, and drawings.

As a technical means for achieving the above-mentioned technical problem, the first aspect of the present application provides a compound represented by Formula 1 below.

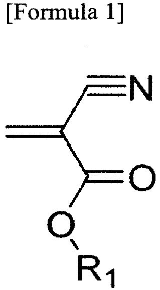

(In Formula 1, R₁ is a linear or branched C₁-C₁₀ alkyl in which one or more hydrogen atoms are substituted with iodine atoms).

In the case of cyanoacrylate-based liquid embolic materials, which are currently widely used as embolic materials, there is a problem in that, since there are not their own radiopacities and thus it is essential to mix them with radiopaque oils before use, it is difficult to secure reproducibility depending on the operator. Moreover, when mixing, the liquid embolic material and the contrast agent are not sufficiently mixed or phase separation occurs so that there is a risk of causing the occurrence of a difference between the blood vessels where the actual embolization occurs and the area contrasted, which causes the occlusion of normal blood vessels not target lesional blood vessels so that there is a problem that can lead to serious medical accidents such as necrosis of normal tissues.

The compound according to the present application has an advantage of having its own radiopacities by containing an iodine atom. Therefore, since pre-mixing of the contrast agent is not necessary, it can be usefully used as an embolic material that can secure reproducibility operator-independently.

According to one embodiment of the present application, the compound may be a compound represented by any one structural formula of Formulas 2 to 4 below, but is not limited thereto.

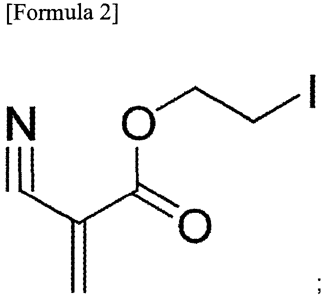

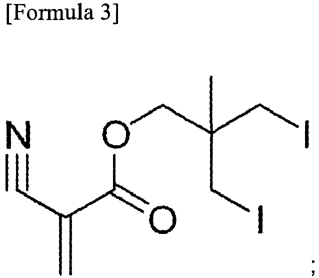

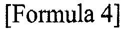

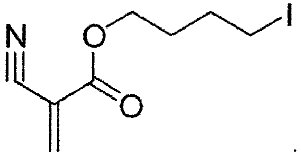

Specifically, Reaction Formula 1 below shows a method for preparing a compound represented by the structural formula of Formula 2 above.

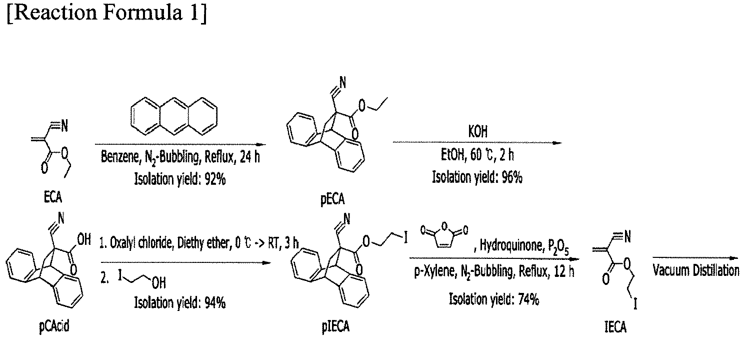

Specifically, Reaction Formula 2 below shows a method for preparing a compound represented by the structural formula of Formula 3 above.

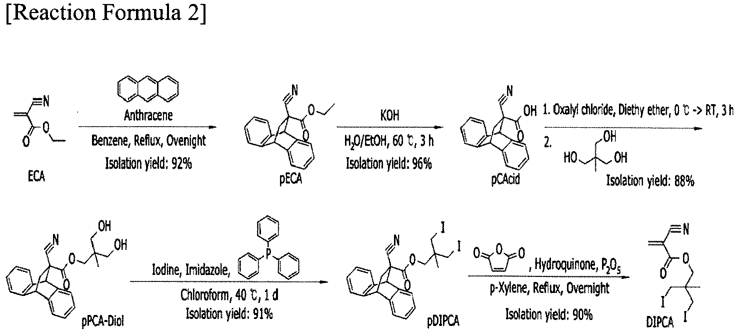

Specifically, Reaction Formula 3 below shows a method for preparing a compound represented by the structural formula of Formula 4 above.

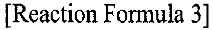

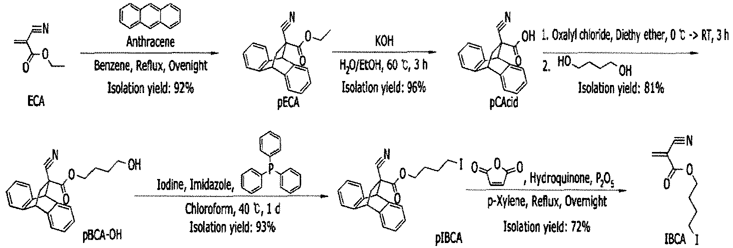

According to one embodiment of the present application, R₁ may be a linear or branched C₁-C₁₀ alkyl in which one or more hydrogen atoms are substituted with fluorine atoms, but is not limited thereto.

In the case of the cyanoacrylate-based liquid embolic materials, which are currently widely used as embolic materials, there is a problem in that the risk of catheter adhesion within the blood vessel is high since the adhesion force is excessively strong.

The compound according to the present application has an advantage of relieving adhesion to the catheter by containing a fluorine atoms. Therefore, it can be usefully used as an embolic material that solves the problem of intravascular catheter adhesion.

According to one embodiment of the present application, the compound may be a compound represented by any one structural formula of Formulas 5 and 6 below, but is not limited thereto.

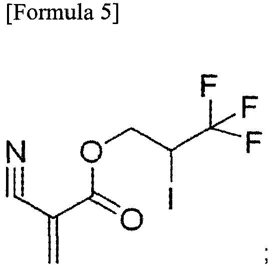

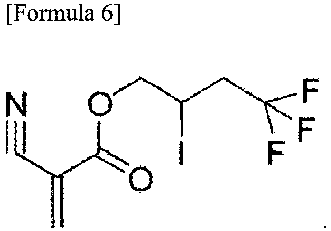

Specifically, Reaction Formula 4 below shows a method for preparing the compound represented by the structural formula of Formula 5 above.

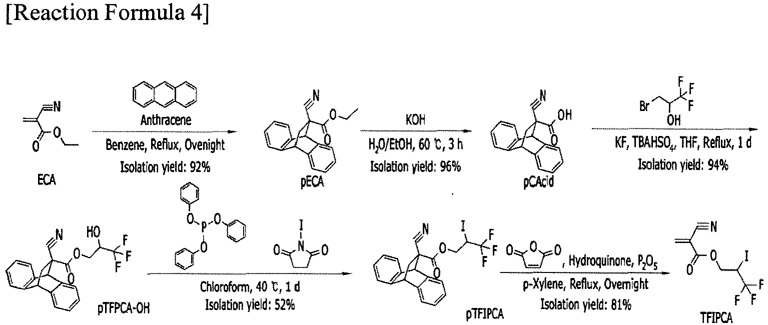

Specifically, Reaction Formula 5 below shows a method for preparing the compound represented by the structural formula of Formula 6 above.

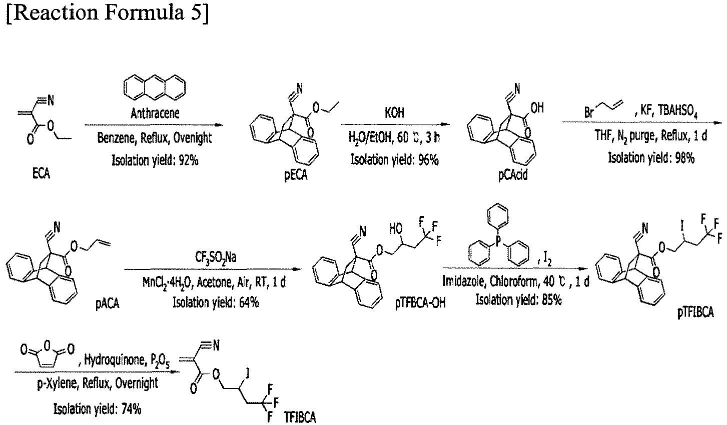

According to one embodiment of the present application, R₁ may be one in which one or more hydrogen atoms are substituted with a substituent represented by Formula 7 below, but is not limited thereto.

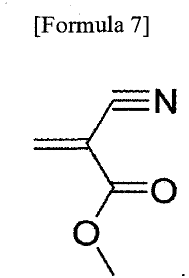

The compound according to the present application has mechanical strength capable of withstanding blood flow by forming a polymer compound. Therefore, it may be usefully used as an embolic material.

According to one embodiment of the present application, the compound may be one in which the substituent represented by Formula 7 above forms a symmetrical structure, but is not limited thereto.

According to one embodiment of the present application, the compound may be compounds represented by Formulas 8 to 9 below, but is not limited thereto.

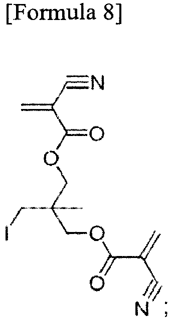

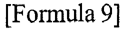

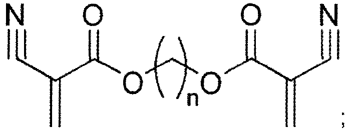

(In Formula 9, n is 1 to 15 linear or branched alkyls).

Specifically, Reaction Formula 6 below shows a method for preparing a compound represented by the structural formula of Formula 8 above.

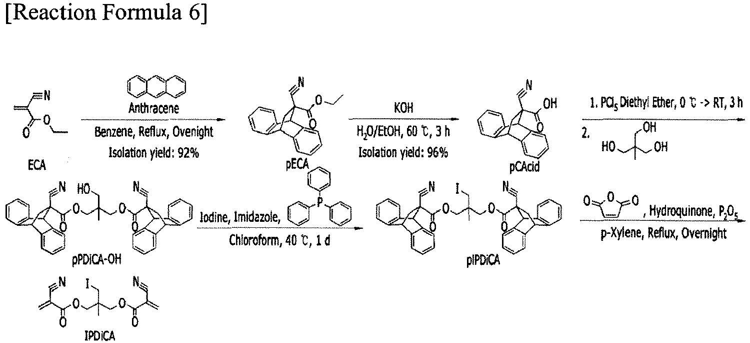

Specifically, Reaction Formula 7 below shows a method for preparing a compound represented by the structural formula of Formula 9 above.

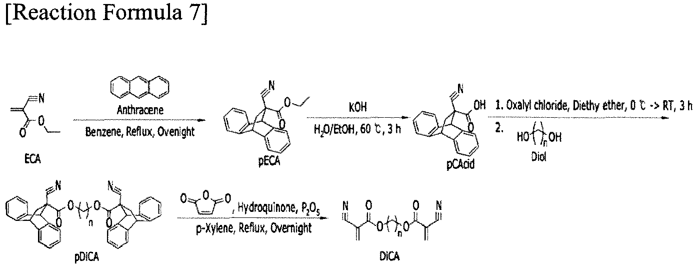

The second aspect of the present application provides a composition for an embolic material, comprising the compound according to the first aspect of the present application and a fatty acid ester oil.

With respect to the composition for an embolic material of the second aspect of the present application, although detailed descriptions of parts overlapping with the first aspect of the present application have been omitted, even if the descriptions have been omitted, the contents described in the first aspect of the present application may be equally applied to the second aspect of the present application.

In the case of cyanoacrylate-based liquid embolic materials currently widely used as embolic materials, the solidification rate is excessively fast so that target lesional blood vessels cannot be accurately occluded, and there is a problem of having a concern that the catheter may be blocked due to their polymerization inside the catheter during the procedure.

The composition for an embolic material according to the present application has an advantage in that the solidification rate is appropriately adjusted by comprising a fatty acid ester oil. Therefore, it may be usefully used as an embolic material capable of accurately occluding target lesional blood vessels.

Further, in the case of embolic materials composed of polymers dissolved in an organic solvent, the solidification rate is low and the adhesion to the catheter is relieved compared to the cyanoacrylate-based liquid embolic materials, but there are problems in that they do not have their own radiopacities and there is a risk of causing toxicity and side effects due to the organic solvent.

The composition for an embolic material according to the present application may be usefully used as an embolic material free of the risk of the occurrence of toxicity and side effects caused by the organic solvent by not using an organic solvent.

According to one embodiment of the present application, the fatty acid ester oil may be a saturated or unsaturated fatty acid ester oil including one selected from the group consisting of a C₄-C₈ lower fatty acid ester oil, a C₈-C₁₂ medium fatty acid ester oil, a C₁₄-C₂₆ higher fatty acid ester oil, and combinations thereof, but is not limited thereto.

According to one embodiment of the present application, the fatty acid ester oil may include one selected from the group consisting of ethyl palmitate, ethyl oleate, ethyl linoleate, ethyl stearate, ethyl decanoate, lipiodol, and combinations thereof, but is not limited thereto.

According to one embodiment of the present application, the compound and the fatty acid ester oil may be contained at a volume ratio of about 10:1 to about 1:10, but is not limited thereto.

The iodine content of the composition for an embolic material and the polymerization rate upon blood contact may be adjusted to an appropriate level by controlling the volume ratio of the fatty acid ester oil.

For example, the compound and the fatty acid ester oil may be contained at a volume ratio of about 3:1 to about 1:3, but is not limited thereto.

According to one embodiment of the present application, the composition for an embolic material may further comprise a compound represented by Formula 10 below, but is not limited thereto.

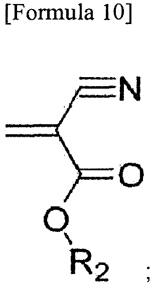

(In Formula 10, R₂ is a linear or branched C₁-C₁₀ alkyl in which one or more hydrogen atoms are substituted with fluorine atoms).

According to one embodiment of the present application, R₂ may include one selected from the group consisting of 2-fluoroethyl, 3-fluoroethyl, 2,2,2-trifluoroethyl, 3,3,3-trifluoropropyl, 2,2,3,3,3-pentafluoropropyl, and combinations thereof, but is not limited thereto.

Specifically, Reaction Formula 8 below shows a method for preparing the compound represented by the structural formula of Formula 10 above.

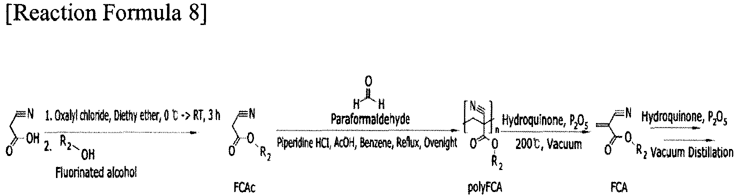

According to one embodiment of the present application, the compound represented by Formula 10 above may be contained at a volume ratio of about 10% to about 50%, but is not limited thereto.

According to one embodiment of the present application, the composition for an embolic material may have an iodine content of about 100 mgl/mL to about 1,500 mgI/mL, but is not limited thereto.

When the composition for an embolic material has an iodine content of about 100 mgl/mL or more, its own radiopacities with respect to X-ray equipment may be secured.

According to one embodiment of the present application, the composition for an embolic material may further comprise one selected from the group consisting of inhibitors, accelerators, and combinations thereof to control the solidification rate, but is not limited thereto.

According to one embodiment of the present application, the inhibitor may be contained at a weight ratio of about 1% to about 30%, but is not limited thereto.

According to one embodiment of the present application, the inhibitor may include one selected from the group consisting of carboxylic acid-containing small molecular substances, acetic acid, phosphoric acid, sulfur dioxide, hydroquinone, carboxylic acid-containing fatty acids, and combinations thereof, but is not limited thereto.

According to one embodiment of the present application, the carboxylic acid-containing fatty acids may include one selected from the group consisting of oleic acid, linolenic acid, palmitic acid, and combinations thereof, but are not limited thereto.

According to one embodiment of the present application, the accelerator may be contained at a weight ratio of about 10% to about 30%, but is not limited thereto.

According to one embodiment of the present application, the accelerator may include one selected from the group consisting of hydroxyl group-containing small molecular substances, ethyl alcohol, butyl alcohol, 1,4-butanediol, and combinations thereof, but is not limited thereto.

According to one embodiment of the present application, the composition for an embolic material may have a solidification rate of about 1 second to about 30 seconds, but is not limited thereto.

The solidification rate of the composition for an embolic material may be adjusted within an error range of about 1 second, but is not limited thereto.

In the case of cyanoacrylate-based liquid embolic materials currently widely used as embolic materials, the solidification rate is excessively fast so that target lesional blood vessels cannot be accurately occluded, and there is a problem of having a concern that the catheter may be blocked due to their polymerization inside the catheter during the procedure.

The composition for an embolic material according to the present application has an advantage in that the solidification rate is appropriately adjusted by comprising a fatty acid ester oil. Therefore, it may be usefully used as an embolic material capable of accurately occluding target lesional blood vessels.

Hereinafter, the present invention will be described in more detail through Examples, but the following Examples are for explanation purposes only and are not intended to limit the scope of the present application.

### [Example 1] Preparation of Compound (IECA) according to First Aspect of the Present Application

The compound (IECA) according to the first aspect of the present application was prepared according to Reaction Formula 1 below.

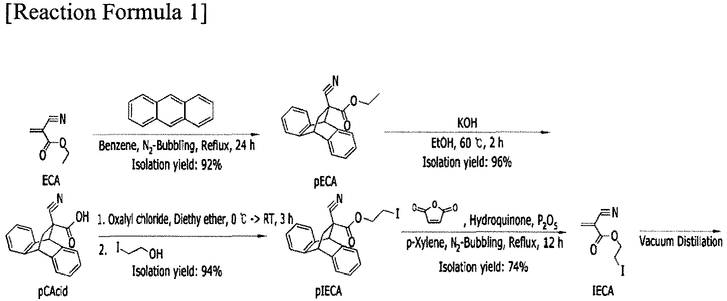

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to modify the end of a carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, an esterification reaction was performed using carboxylic acid of pCAcid and an alkyl alcohol containing iodine. Carboxylic acid of pCAcid was activated with acyl chloride using oxalyl chloride for an esterification reaction, and then reacted with an iodinated alkyl alcohol to synthesize a protected cyanoacrylate to which the iodinated alkyl ester was bonded. After completion of the reaction, a product in which an iodinated alkyl group was bonded to the end of pCA (pIECA) was obtained, and the product was separated using column chromatography.

Subsequently, a reaction between maleic anhydride and pIECA was performed under reflux conditions using para-xylene as a solvent, thereby removing the protecting group. After the reaction was completed, a product (IECA) was obtained by performing distillation under reduced pressure.

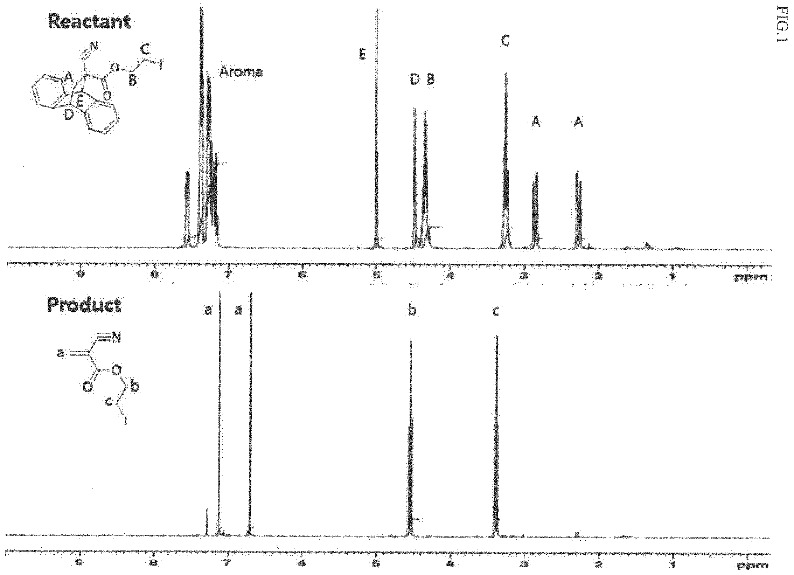

FIG. 1 is a nuclear magnetic resonance (NMR) spectrum of the compound (IECA) prepared in Example 1 of the present application.

It could be confirmed through FIG. 1 that Compound 1 below was actually prepared.

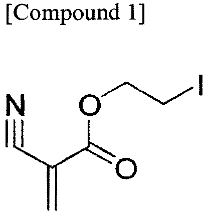

### [Example 2] Preparation of Compound (DIPCA) according to First Aspect of the Present Application

A compound (DIPCA) according to the first aspect of the present application was prepared according to Reaction Formula 2 below.

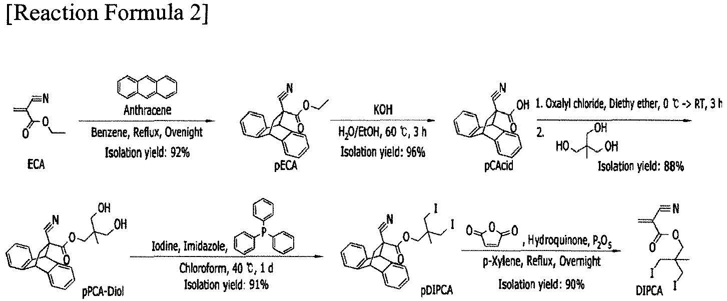

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to modify the end of a carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, an esterification reaction using carboxylic acid of pCAcid and tris(hydroxymethyl)ethane (triol) containing three alcohol groups was performed. Carboxylic acid of pCAcid was activated with acyl chloride using oxalyl chloride for an esterification reaction, and then reacted with a triol to synthesize protected cyanoacrylate (pPCA-Diol) to which one molecule of triol was bonded. After completion of the reaction, a product containing two alcohol groups at the ends of pCA was obtained, and the product was separated using column chromatography.

Subsequently, a reaction of substituting two alcohol groups in pPCA-Diol with iodine was performed. After dissolving pPCA-Diol in anhydrous chloroform, imidazole, triphenylphosphine, and iodine were sequentially quantified, put thereinto, and heated to 40°C to proceed with the reaction. After the reaction was completed, a product (pDIPCA) was separated using column chromatography.

Subsequently, a reaction between maleic anhydride and anthracene was performed under reflux conditions using para-xylene as a solvent, and thus the protecting group was removed. After the reaction was completed, para-xylene was evaporated, and the reaction product (DIPCA) having para-xylene evaporated therefrom was precipitated in anhydrous benzene three times to remove impurities.

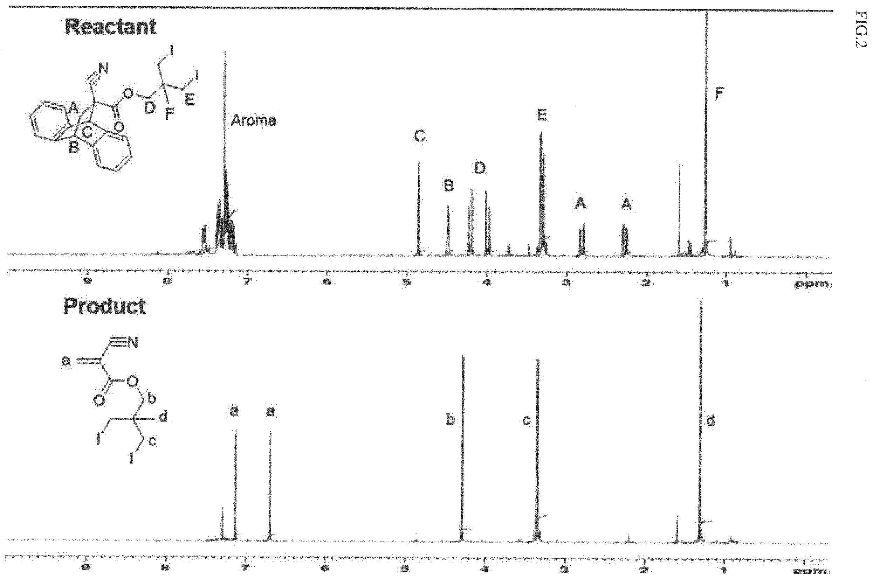

FIG. 2 is a nuclear magnetic resonance (NMR) spectrum of the compound (DIPCA) prepared in Example 2 of the present application.

It could be confirmed through FIG. 2 that Compound 2 below was actually prepared.

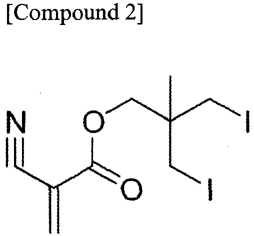

### [Example 3] Preparation of Compound (IBCA) according to First Aspect of the Present Application

The compound (IBCA) according to the first aspect of the present application was prepared according to Reaction Formula 3 below.

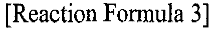

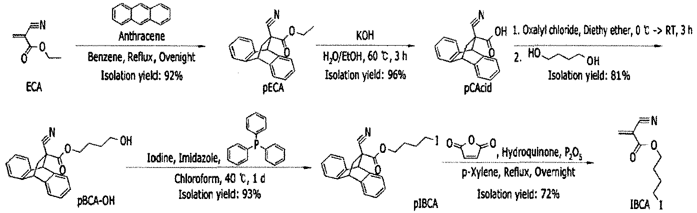

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to more easily introduce an iodinated group at the end of the carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, an esterification reaction using carboxylic acid of pCAcid and 1,4-butanediol (diol) containing two alcohol groups was performed. Carboxylic acid of pCAcid was activated with acyl chloride using oxalyl chloride for an esterification reaction, and then reacted with a diol to synthesize protected cyanoacrylate (pBCA-OH) to which a hydroxybutyl group was bonded. After completion of the reaction, a product containing one alcohol group at the end of pCA was obtained, and the product was separated using column chromatography.

Subsequently, a reaction of substituting two alcohol groups in pBCA-OH with iodine was performed. After dissolving pBCA-OH in anhydrous chloroform, imidazole, triphenylphosphine, and iodine were sequentially quantified, put thereinto, and heated to 40°C to proceed with the reaction. After the reaction was completed, a product (pIBCA) was separated using column chromatography.

Subsequently, a reaction between maleic anhydride and anthracene was performed under reflux conditions using para-xylene as a solvent, and thus the protecting group was removed. After the reaction was completed, a product (IBCA) was obtained by performing distillation under reduced pressure.

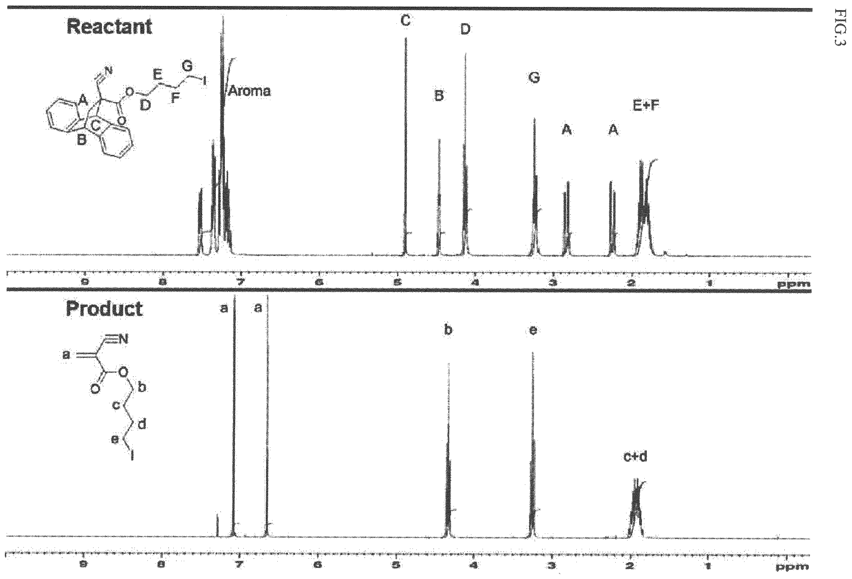

FIG. 3 is a nuclear magnetic resonance (NMR) spectrum of the compound (IBCA) prepared in Example 3 of the present application.

It could be confirmed through FIG. 3 that Compound 3 below was actually prepared.

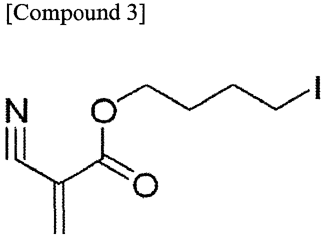

### [Example 4] Preparation of Compound (TFIPCA) according to First Aspect of the Present Application

The compound (TFIPCA) according to the first aspect of the present application was prepared according to Reaction Formula 4 below.

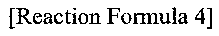

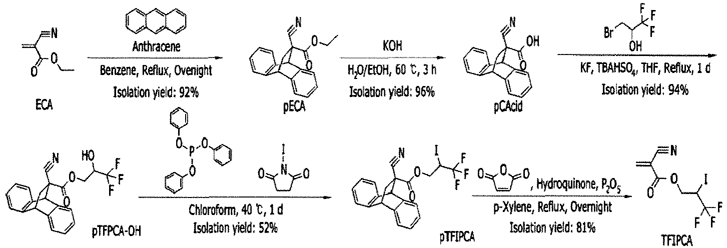

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to modify the end of a carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, carboxylic acid of pCAcid was activated using anhydrous potassium fluoride, and then esterification was induced through reaction with a bromo group in bromofluoro alcohol under reflux conditions. After the reaction was completed, protected fluoroalkyl alcohol cyanoacrylate (pTFPCA-OH) was separated using column chromatography.

Subsequently, a substitution reaction using triphenyl phosphite and N-iodosuccinimide was performed at a temperature of 40°C using chloroform as a solvent for one day. After the reaction was completed, protected fluoro-iodinated cyanoacrylate (pTFIPCA) was separated using column chromatography.

Subsequently, a reaction between maleic anhydride and anthracene was performed under reflux conditions using para-xylene as a solvent, and thus the protecting group was removed. After the reaction was completed, a product (TFIPCA) was obtained by performing distillation under reduced pressure.

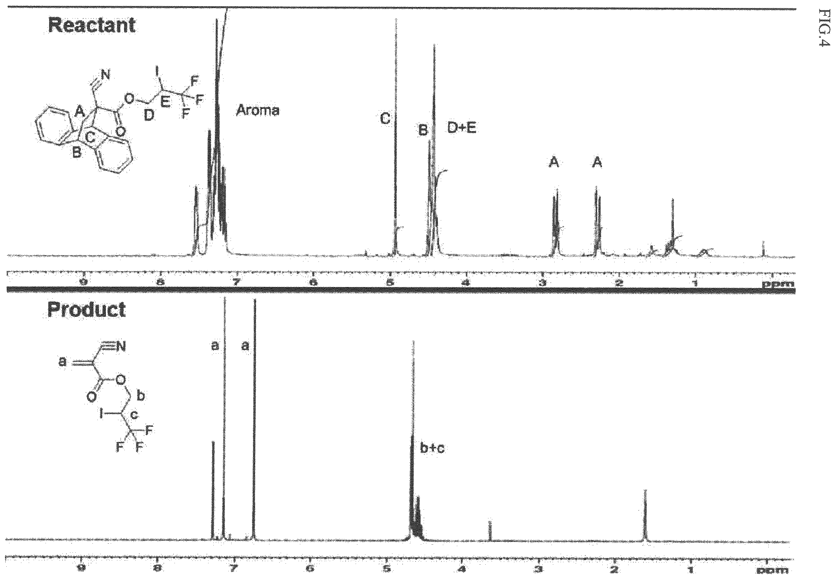

FIG. 4 is a nuclear magnetic resonance (NMR) spectrum of the compound (TFIPCA) prepared in Example 4 of the present application.

It could be confirmed through FIG. 4 that Compound 4 below was actually prepared.

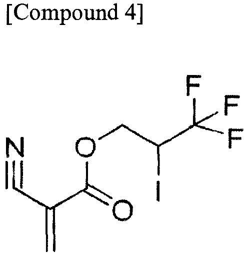

### [Example 5] Preparation of Compound (TFIBCA) according to First Aspect of the Present Application

The compound (TFIBCA) according to the first aspect of the present application was prepared according to Reaction Formula 5 below.

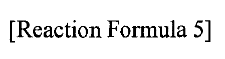

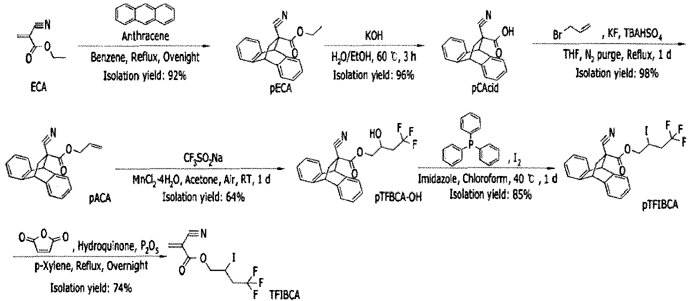

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to modify the end of a carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, carboxylic acid of pCAcid was activated using anhydrous potassium fluoride, and then esterification was induced through reaction with a bromo group in allyl bromide under reflux conditions. After the reaction was completed, protected allyl cyanoacrylate (pACA) was separated using column chromatography.

Subsequently, trifluoromethyl radicals were formed using trifluoromethylsulfmate ions using divalent manganese cations as a catalyst. The formed radicals were reacted with an alkene group contained in pACA at room temperature (15°C to 25°C) to proceed with an oxyfluoromethylation reaction. The formed radicals were reacted with the alkene group of pACA. After the reaction was completed, protected fluoroalkyl alcohol cyanoacrylate (pTFBCA-OH) was separated using column chromatography.

Subsequently, a substitution reaction using triphenyl phosphite and N-iodosuccinimide was performed at a temperature of 40°C using chloroform as a solvent for one day. After the reaction was completed, protected fluoro-iodinated cyanoacrylate (pTFIBCA) was separated using column chromatography.

Subsequently, a reaction between maleic anhydride and anthracene was performed under reflux conditions using para-xylene as a solvent, and thus the protecting group was removed. After the reaction was completed, a product (TFIPBCA) was obtained by performing distillation under reduced pressure.

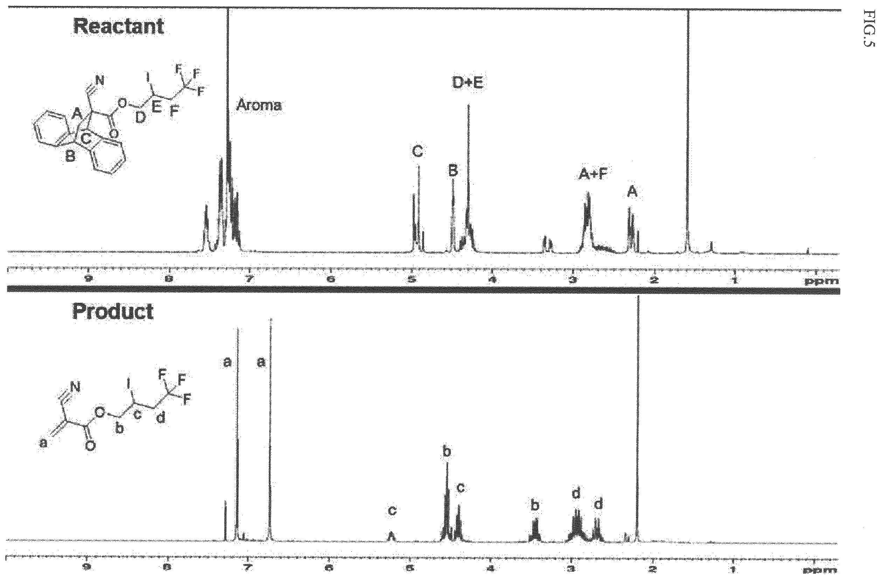

FIG. 5 is a nuclear magnetic resonance (NMR) spectrum of the compound (TFIBCA) prepared in Example 5 of the present application.

It could be confirmed through FIG. 5 that Compound 5 below was actually prepared.

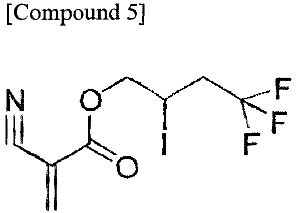

### [Example 6] Preparation of Compound (IPDiCA) according to First Aspect of the Present Application

The compound (IPDiCA) according to the first aspect of the present application was prepared according to Reaction Formula 6 below.

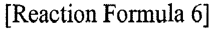

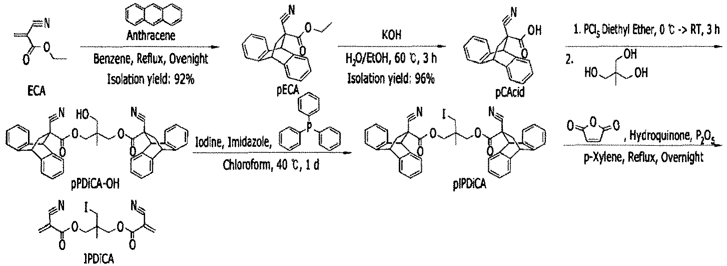

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to modify the end of a carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, an esterification reaction using carboxylic acid of pCAcid and tris(hydroxymethyl)ethane (triol) containing three alcohol groups was performed. Carboxylic acid of pCAcid was activated with acyl chloride using oxalyl chloride for an esterification reaction, and then reacted with a triol to synthesize a product (pPDiCA-OH) in which two molecules of protected cyanoacrylate were bonded to one molecule of triol. After completion of the reaction, a product containing two pCA and one alcohol group in one molecule was obtained, and the product was separated using column chromatography.

Subsequently, a reaction of substituting the alcohol group contained in pPDiCA-OH with iodine was performed. After dissolving pPDiCA-OH in anhydrous chloroform, imidazole, triphenylphosphine, and iodine were sequentially quantified, put thereinto, and heated to 40°C to proceed with the reaction. After the reaction was completed, the product (pIPDiCA) was separated using column chromatography.

Subsequently, a reaction between maleic anhydride and anthracene was performed under reflux conditions using para-xylene as a solvent, and thus the protecting group was removed. After the reaction was completed, para-xylene was evaporated, and the reaction product (IPDiCA) having para-xylene evaporated therefrom was precipitated in anhydrous benzene three times to remove impurities.

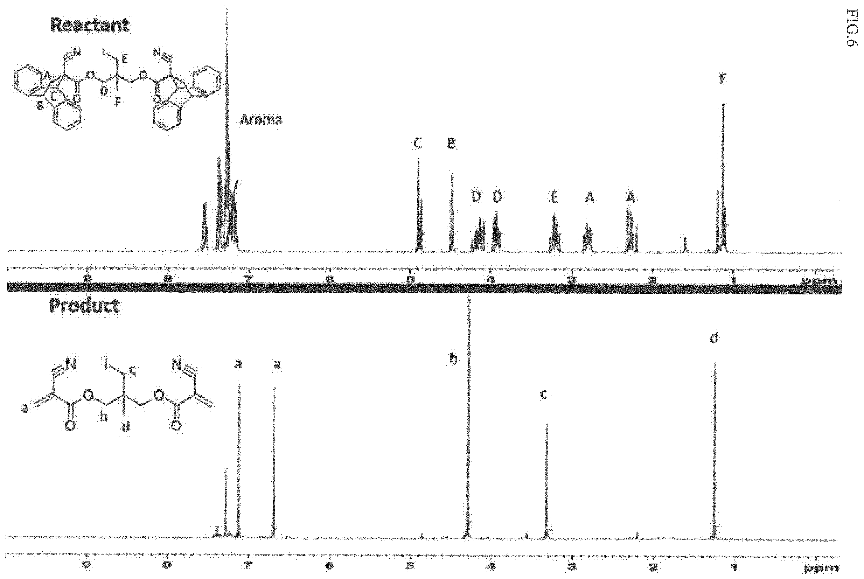

FIG. 6 is a nuclear magnetic resonance (NMR) spectrum of the compound (IPDiCA) prepared in Example 6 of the present application.

It could be confirmed through FIG. 6 that Compound 6 below was actually prepared.

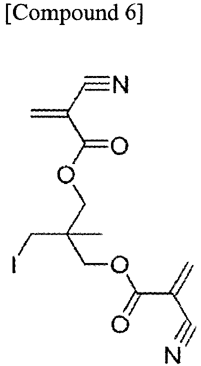

### [Example 7] Preparation of Compound (DiCA) according to First Aspect of the Present Application

The compound (DiCA) according to the first aspect of the present application was prepared according to Reaction Formula 7 below.

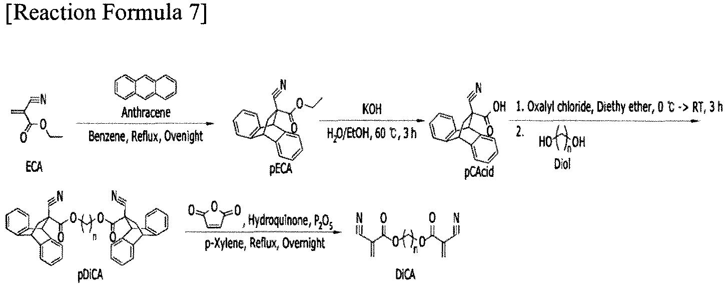

Specifically, anthracene and ethyl 2-cyanoacrylate were refluxed under a nitrogen atmosphere using anhydrous benzene as a solvent to perform a reaction for protecting a double bond group of cyanoacrylate. After the reaction was completed, the resulting solution was cooled to room temperature (15°C to 25°C), residual anthracene was filtered, and then the solvent was evaporated to obtain a product.

Subsequently, in order to modify the end of a carbonyl group of protected cyanoacrylate (pCA), the carbonyl group was hydrolyzed to obtain a product having a carboxylic acid form (protected cyanoacrylic acid; pCAcid). The hydrolysis reaction of the carbonyl group was carried out by performing heating at 60°C under basic conditions using water as a co-solvent in ethanol, and after the reaction was completed, the product was separated by performing extraction with an aqueous acid solution-diethyl ether.

Subsequently, an esterification reaction using carboxylic acid of pCAcid and a diol containing two alcohol groups was performed. Carboxylic acid of pCAcid was activated with acyl chloride using oxalyl chloride for an esterification reaction, and then reacted with a diol to synthesize a product (pDiCA) in which two molecules of protected cyanoacrylate were bonded to one molecule of diol. After completion of the reaction, a product (pDiCA) containing two molecules of protected cyanoacrylate and a linear or branched alkyl group in one molecule was obtained, and the product was separated using column chromatography.

Subsequently, a reaction between maleic anhydride and anthracene was performed under reflux conditions using para-xylene as a solvent, and thus the protecting group was removed. After the reaction was completed, para-xylene was evaporated, and the reaction product (DiCA) having para-xylene evaporated therefrom was precipitated in anhydrous benzene three times to remove impurities.

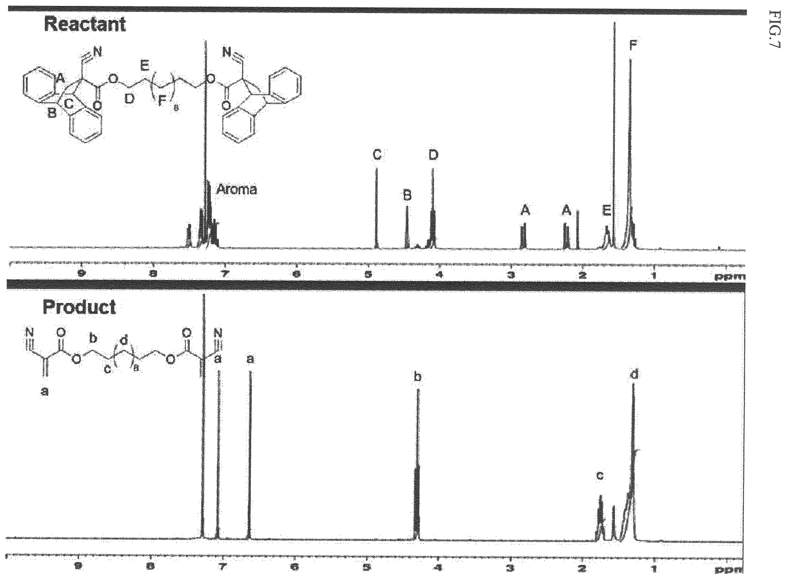

FIG. 7 is a nuclear magnetic resonance (NMR) spectrum of the compound (DiCA) prepared in Example 7 of the present application.

It could be confirmed through FIG. 7 that Compound 7 below was actually prepared.

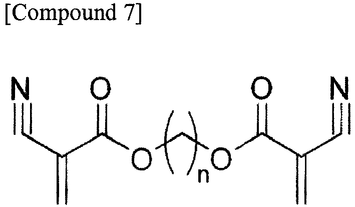

### [Example 8] Preparation of Fluorinated Cyanoacrylate Compound (FCA)

A fluorinated cyanoacrylate compound (FCA) was prepared according to Reaction Formula 8 below.

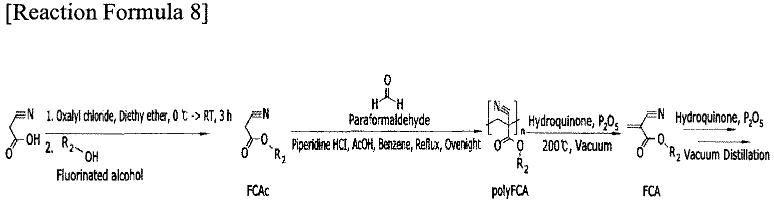

Specifically, an esterification reaction between carboxylic acid of cyanoacetic acid and an alcohol containing fluorine was performed. Carboxylic acid of cyanoacetic acid was activated with acyl chloride using oxalyl chloride for an esterification reaction, and then reacted with fluorinated alcohols to synthesize cyanoacetate containing a fluoroalkyl group. Afterwards, a product was separated using distillation under reduced pressure.

The fluorinated alcohols (R₂-OH) used in the synthesis correspond to 2-fluoroethanol, 3-fluoropropanol, 2,2,2-trifluoroethanol, 3,3,3-trifluoropropanol, and 2,2,3,3,3-pentafluoropropanol, and synthesis methods using the respective fluorinated alcohols were equally performed.

Subsequently, fluoroalkyl cyanoacetate was reacted with formaldehyde to form a fluoroalkyl cyanoacrylate polymer. The polymer synthesis reaction was carried out under reflux conditions by dissolving fluoroalkyl cyanoacetate and paraformaldehyde in a benzene solvent and then using a piperidine salt as a catalyst under reflux conditions. After evaporating the benzene solvent from the resulting solution, the crude product was redissolved in acetone and filtered to remove residual paraformaldehyde. Afterwards, the crude product was precipitated in diethyl ether to obtain pure fluoroalkyl cyanoacrylate polymer.

Subsequently, fluoroalkyl cyanoacrylate was obtained by thermal depolymerization of fluoroalkyl cyanoacrylate polymer. The depolymerization reaction was performed by adding phosphorus pentoxide and hydroquinone as repolymerization inhibitors to the fluoroalkyl cyanoacrylate polymer, and then heating the mixture to 200°C. A depolymerized product, fluoroalkyl 2-cyanoacrylate (FCA), was obtained by performing distillation under reduced pressure.

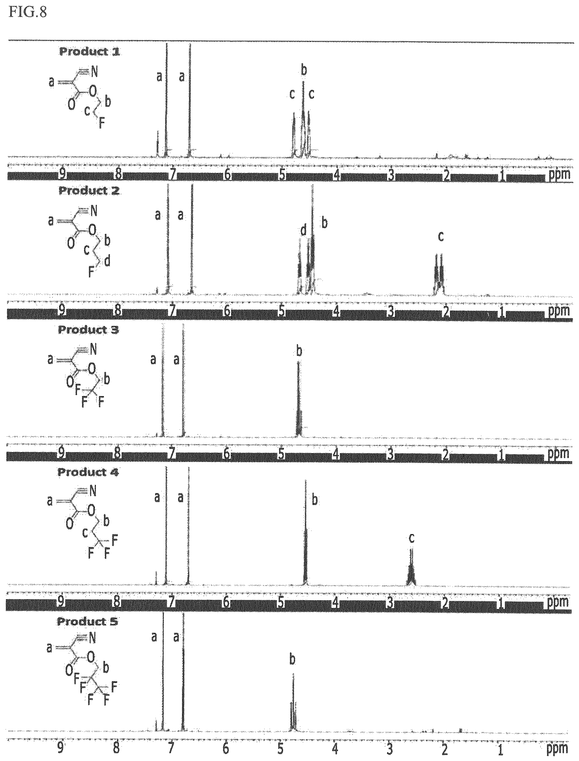

FIG. 8 is nuclear magnetic resonance (NMR) spectra of the compounds (FCA) prepared in Example 8 of the present application.

It could be confirmed through FIG. 8 that Compound 8 below was actually prepared.

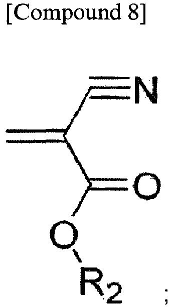

(In Compound 8, the fluorinated alcohols (R₂-OH) used for R₂ synthesis correspond to 2-fluoroethanol, 3-fluoropropanol, 2,2,2-trifluoroethanol, 3,3,3-trifluoropropanol, and 2,2,3,3,3-pentafluoropropanol).

### [Example 9] Preparation of Compositions for Embolic Material according to Second Aspect of the Present Application

The compounds prepared in Examples 1 to 8 above and ethyl oleate were mixed at volume ratios according to Table 1 below to prepare compositions for an embolic material, and the compositions for an embolic material were named as S1 to S5.

**[Table 1]**

| Classification | Volume ratio (%) |
|---|---|
| S1 | IECA (30) + TFECA (40) + Ethyl oleate (30) |
| S2 | IECA (20) + PFPCA (10) + Ethyl oleate (70) |
| S3 | IBCA (40) + Ethyl oleate (59) + Acetic acid (1) |
| S4 | IECA (15) + IBCA (30) + Ethyl oleate (55) |
| S5 | TFIPCA + Ethyl oleate |

### [Comparative Example]

Compositions prepared by mixing commercially available Histoacryl (H), Lipiodol, and a combination thereof at volume ratios according to Table 2 below were used as those of Comparative Example, and the mixed compositions were named HL1 to HL4.

**[Table 2]**

| Classification | Volume ratio (%) |
|---|---|
| HL1 | Histoacryl (50) + Lipiodol (50) |
| HL2 | Histoacryl (33) + Lipiodol (67) |
| HL3 | Histoacryl (25) + Lipiodol (75) |
| HL4 | Histoacryl (20) + Lipiodol (80) |

### [Experimental Example 1]

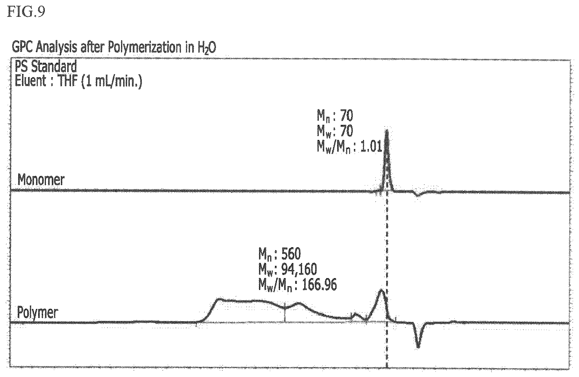

FIG. 9 is an image showing the result of gel permeation chromatography (GPC) analysis of the compound prepared in one Example of the present application.

Specifically, 50 uL of the monomer of the compound (IECA) prepared in Example 1 above was dropped into 4 mL of double distilled water, and then allowed to stand until solidification (polymerization) proceeded completely. After solidification was completed, the solidified compound was obtained by performing filtration and dry for the GPC analysis.

It could be confirmed through this that polymerization and solidification of the compound (IECA) prepared in Example 1 above normally occurred upon contact with moisture.

### [Experimental Example 2]

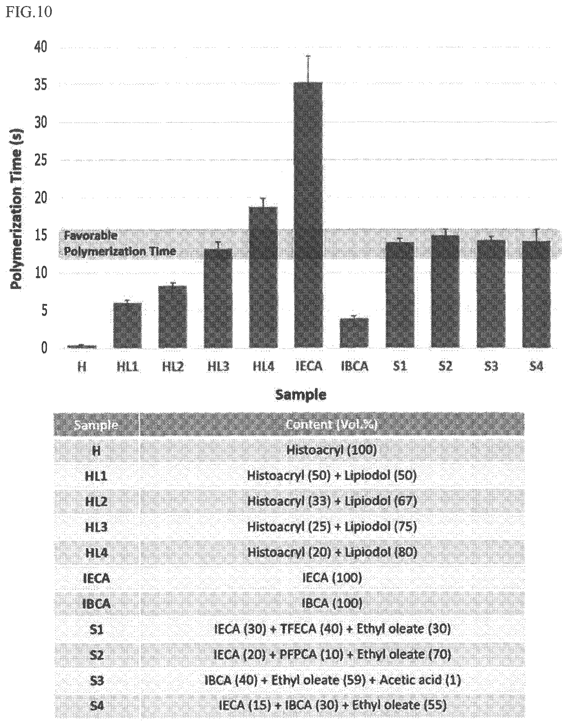

FIG. 10 is a graph showing the results of measuring the times required for polymerization and solidification of the compounds prepared in one Example of the present application upon contact with blood.

Specifically, 20 mL of pig blood was collected, treated with EDTA to prevent coagulation of blood, and then centrifuged to 10,000 rpm at 4°C for 15 minutes to separate plasma components. After dropping 50 uL of the separated plasma components on a slide glass, the compound (IECA) prepared in Example 1 above, the compound (IBCA) prepared in Example 2, the compounds (S1 to S4) prepared in Example 9, and Comparative Example (H and HL1 to HL4) were dropped thereon in an amount of 20 uL and brought into contact with plasma components. Times from immediately after contact were measured, and thus the times required until solidification was completely completed and the solid material became opaque were checked. After repeating the same experiment 3 times for each sample, the average of the required times was calculated.

It could be confirmed through this that the solidification times of the compound (IECA) prepared in Example 1, the compound (IBCA) prepared in Example 2, and the compounds (S1 to S4) prepared in Example 9 could be adjusted to a level similar to that of Comparative Example.

### [Experimental Example 3]

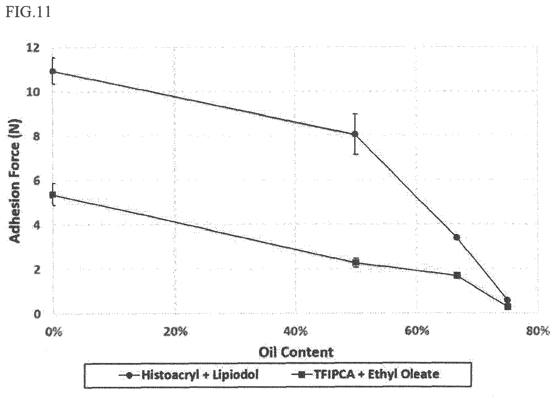

FIG. 11 is a graph showing the results of measuring the catheter adhesion according to oil (ethyl oleate) contents of the compounds prepared in one Example of the present application.

The catheter adhesion was measured while changing the oil (ethyl oleate or lipiodol) contents of the compound (S5) according to Example 9 above and the compound (Histoacryl + Lipiodol) according to Comparative Example.

Specifically, after preparing a polypropylene frame having a diameter of 5 mm and a length of 30 mm, 0.25 mL of pig blood was injected into the frame. The compounds prepared in Examples 1 to 9 were injected into pig blood in an amount of 0.20 mL through a microcatheter, and solidification of the compounds was induced in a state in which the tip of the catheter was submerged in blood. After drying the prepared specimen for 1 day, a tensile test was performed at a speed of 1 mm/min through a tensile tester so that the force required to retrieve the catheter from the inside of the compound solidified along with blood was measured to confirm the adhesion to the catheter. After repeating the same experiment 3 times for each sample, the average of the adhesion was calculated.

It could be confirmed through this that the adhesion of the compound (S5) prepared in Example 9 above to the catheter could be adjusted to a level similar to that of Comparative Example.

### [Experimental Example 4]

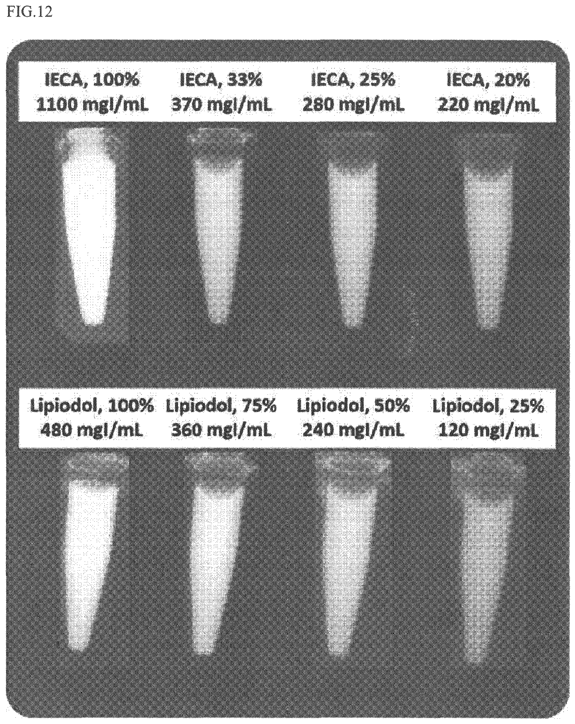

FIG. 12 is X-ray images showing the results of measuring the radiopacities under in vitro conditions of the compounds according to one Example and Comparative Example of the present application.

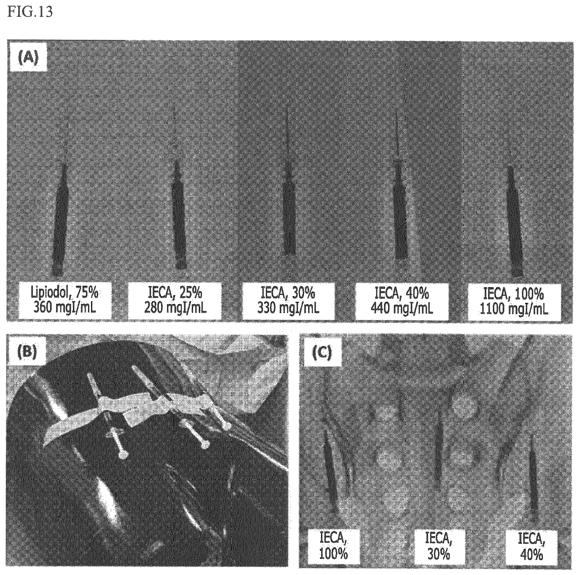

FIG. 13 is images showing the results of measuring the radiopacities using the compounds prepared in one Example of the present application. They were measured using real-time C-arm X-ray devices.

It could be confirmed through this that the compound (IECA) prepared in Example 1 exhibited a level of radiopacities similar to that of Comparative Example (Lipiodol).

### [Experimental Example 5]

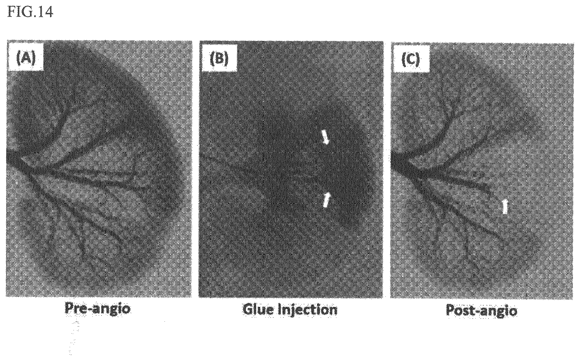

FIG. 14 is images showing the results of measuring the radiopacities and embolic effects under in vivo conditions using a compound prepared in one Example of the present application.

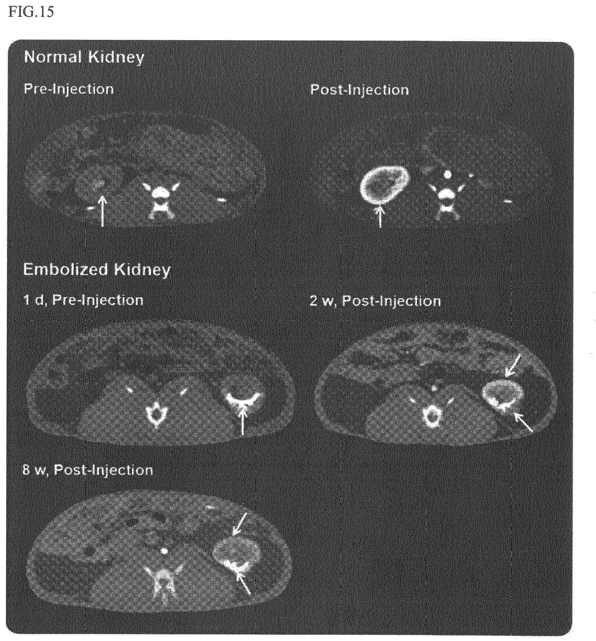

FIG. 15 is CT images of the renal artery taken with the passage of time after performing embolization using a compound prepared in one Example of the present application.

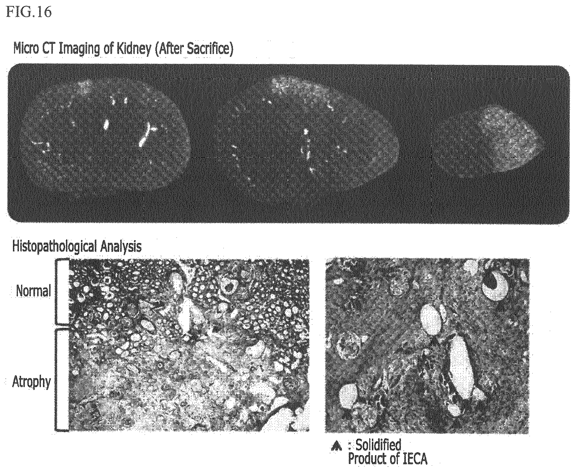

FIG. 16 is CT images and histopathology images taken after performing embolization using a compound prepared in one Example of the present application.

Specifically, after anesthetizing New Zealand white rabbits, a catheter was inserted into the artery located in the rabbits' ears. Thereafter, a catheter was introduced into the renal artery under radioscopy using a guide wire. After checking the shape and position of the renal artery by injecting a radiographic contrast agent through the catheter, the compound (S1) prepared in Example 9 above was injected into the renal artery using a 0.5 mL lock syringe under radioscopy. Subsequently, the radiographic contrast agent was injected again into the renal artery to determine whether the renal artery was embolized or not by checking whether or not the contrast agent was discharged into the bladder through the ureter. The rabbits that had completed renal artery embolization were observed through computed tomography for 8 weeks to check changes in the embolized kidneys, and after the passage of 8 weeks, the rabbits were sacrificed to analyze the kidney tissues.

It could be confirmed through this that the compound (S1) prepared in Example 9 above exhibits the radiopacities and embolic effects with respect to X-ray under in vitro and in vivo conditions.

### [Experimental Example 6]

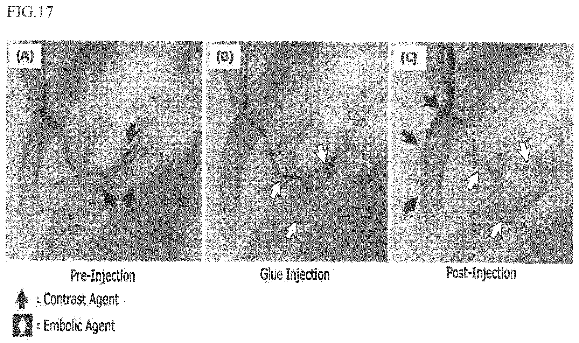

FIG. 17 is images showing the results of measuring the radiopacities and embolic effects under in vivo conditions after performing embolization using a compound prepared in one Example of the present application.

Specifically, after anesthetizing a pig (domestic swine), a catheter was inserted into the artery located in the femoral region, and a catheter was introduced into the intercostal artery or the renal artery under radioscopy using a guide wire. After checking the shape and position of the artery by injecting a radiographic contrast agent through the catheter, the compound (S1) prepared in Example 9 above was injected into the artery using a 0.5 mL lock syringe under radioscopy. Subsequently, the solidification rate within the blood vessel and the adhesion to the catheter were checked under radioscopy. Subsequently, the radiographic contrast agent was injected again to check whether the artery was embolized or not.

It could be confirmed through this that the compound (S1) prepared in Example 9 above exhibits the radiopacities and embolic effects with respect to X-ray under in vivo conditions in a large animal model having organs of a size and shape similar to those of the human body.

The foregoing description of the present application is for illustration, and those with ordinary skill in the art to which the present application pertains will be able to understand that it can be easily modified into other specific forms without changing the technical spirit or essential features of the present application. Therefore, it should be understood that the embodiments described above are illustrative in all respects and not restrictive. For example, each element described as a single form may be implemented in a dispersed form, and likewise elements described in the dispersed form may also be implemented in a combined form.

The scope of the present application is indicated by the claims to be described later rather than the above detailed description, and all changes or modified forms derived from the meaning and scope of the claims and equivalent concepts thereof should be construed as being included in the scope of the present application.

## Claims

1. A compound represented by the following Formula 1: 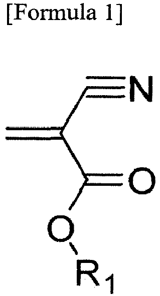 (In Formula 1, R₁ is a linear or branched C₁-C₁₀ alkyl in which one or more hydrogen atoms are substituted with iodine atoms).

2. The compound of claim 1, wherein the compound is represented by any one structural formula of the following Formulas 2 to 4:

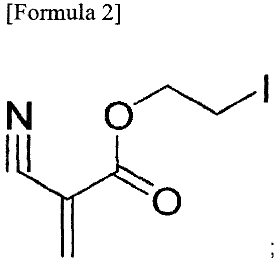

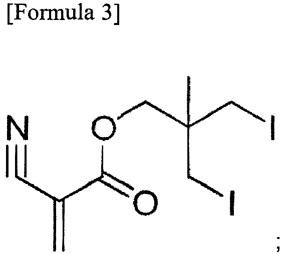

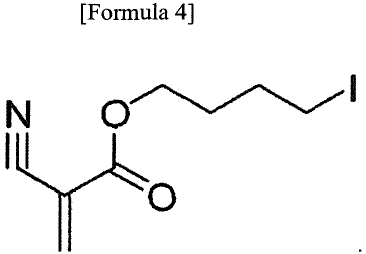

3. The compound of claim 1, wherein R₁ is a linear or branched C₁-C₁₀ alkyl in which one or more hydrogen atoms are substituted with fluorine atoms.

4. The compound of claim 3, wherein the compound is represented by any one structural formula of the following Formulas 5 and 6:

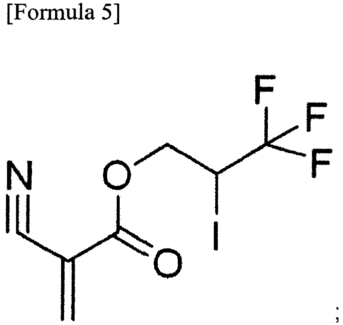

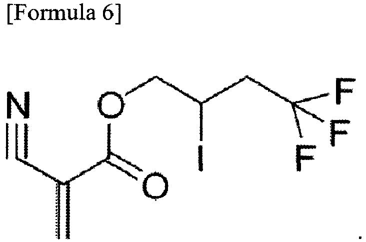

5. The compound of claim 1, wherein R₁ is one in which one or more hydrogen atoms are substituted with a substituent represented by the following Formula 7:

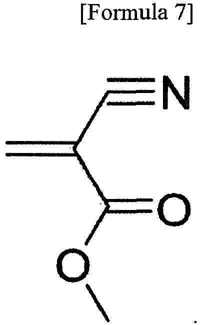

6. The compound of claim 5, wherein the compound is one in which the substituent represented by Formula 7 above forms a symmetrical structure.

7. The compound of claim 5, wherein the compound is represented by any one structural formula of the following Formulas 8 and 9: 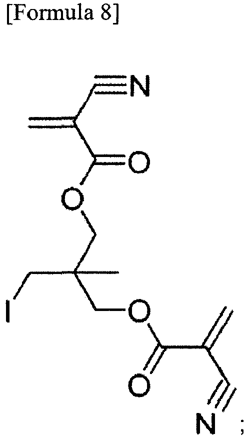 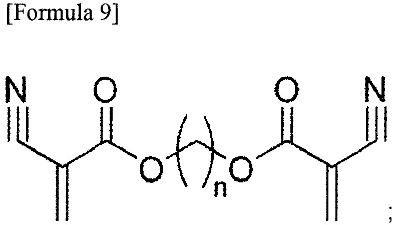 (In Formula 9, n is 1 to 15 linear or branched alkyls).

8. A composition for an embolic material, comprising the compound according to any one of claims 1 to 7 and a fatty acid ester oil.

9. The composition of claim 8, wherein the fatty acid ester oil is a saturated or unsaturated fatty acid ester oil including one selected from the group consisting of a C₄-C₈ lower fatty acid ester oil, a C₈-C₁₂ medium fatty acid ester oil, a C₁₄-C₂₆ higher fatty acid ester oil, and combinations thereof.

10. The composition of claim 8, wherein the fatty acid ester oil includes one selected from the group consisting of ethyl palmitate, ethyl oleate, ethyl linoleate, ethyl stearate, ethyl decanoate, lipiodol, and combinations thereof.

11. The composition of claim 8, wherein the compound and the fatty acid ester oil are contained at a volume ratio of 10:1 to 1:10.

12. The composition of claim 8, wherein the composition for an embolic material further comprises a compound represented by the following Formula 10: 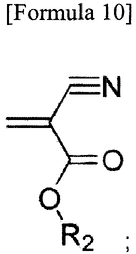 (In Formula 10, R₂ is a linear or branched C₁-C₁₀ alkyl in which one or more hydrogen atoms are substituted with fluorine atoms).

13. The composition of claim 12, wherein R₂ includes one selected from the group consisting of 2-fluoroethyl, 3-fluoroethyl, 2,2,2-trifluoroethyl, 3,3,3-trifluoropropyl, 2,2,3,3,3-pentafluoropropyl, and combinations thereof.

14. The composition of claim 12, wherein the compound represented by Formula 10 above is contained at a volume ratio of 10% to 50%.

15. The composition of claim 8, wherein the composition for an embolic material has an iodine content of 100 mgI/mL to 1,500 mgI/mL.

16. The composition of claim 8, wherein the composition for an embolic material further comprises one selected from the group consisting of inhibitors, accelerators, and combinations thereof to control the solidification rate.

17. The composition of claim 16, wherein the inhibitor is contained at a weight ratio of 1% to 30%.

18. The composition of claim 16, wherein the accelerator is contained at a weight ratio of 10% to 30%.

19. The composition of claim 8, wherein the composition for an embolic material has a solidification rate of 1 second to 30 seconds.

20. The composition of claim 19, wherein the solidification rate of the composition for an embolic material is adjusted within an error range of 1 second.
